# EUROPEAN PATENT APPLICATION

(11) **EP 1 246 077 A1**
(43) Date of publication of application: **02.10.2002**
(21) Application number: 01107524.9
(22) Date of filing: 26.03.2001
(51) Int. Cl.: G06F 17/30, G06F 19/00

(54) **Method and apparatus for structuring and searching sets of signals**

(71) Applicant: LION Bioscience AG, 69120 Heidelberg (DE)
(72) Inventor: Kirsch, Harald, 69123 Heidelberg (DE); Schuhmann, Dietrich, Dr., 69214 Eppelheim (DE)
(74) Representative: Schohe, Stefan

(57) **Abstract**

The invention provides a method of structuring a set of signals according to predetermined patterns by means of an apparatus for processing said signals, said patterns forming a hierarchy, wherein a pattern of a higher order comprises at least one pattern of a lower order, said method comprising the steps of:
- providing a set of signals comprising at least one unit of signals matching a signal pattern, said unit comprising information identifying the signals comprised in said unit,
- comparing a group of signals out of said set of signals, comprising at least one of said units, to one or more predetermined patterns of a higher order than the order of a unit contained in said group of signals by said processing apparatus,
- if a match is found, modifying said set of signals by said processing apparatus, said step of modifying said set comprising the step of replacing the group of signals matching the pattern by a higher order unit corresponding to said group of signals matching said higher order pattern, said unit having an order corresponding to the order of the higher order pattern and comprising information identifying the signals comprised in said unit.

## Description

The invention relates to a method of structuring sets of signals, especially sequences of signals, with the purpose of improving the possibilities of a search for and extraction of information contained in said set. The invention especially relates to structuring text data and/or genetic data.

It is a well known problem in the search of text data that looking for a certain word pattern in a large number of texts may result in large number of hits containing only a small percentage of information relevant to the proper query. Restricting the search to the simultaneous occurrence of certain words with a certain distance to each other may help in some instances, but is still far from satisfactory. As this criterion only relates to the order of the words in a text, but does not take into account their relation or the structure of the text, there is still the problem of getting a high number of non-relevant results, combined with the risk of missing relevant information due to restrictive search criteria.

Another problem is that in many instances information available on databases is not structured at all or not properly structured. This especially relates to information from databases for experimental data, such as, for example, gene sequences or atomic spectra. Taking up these two examples, there is information available virtually forming a fingerprint of an organism or a substance, which can, however, only be exploited by a detailed analysis which may require both a high amount of skill and manpower. Although such analysis is, of course, carried out by specialists using the respective databases, the result is either not communicated to other people or communicated through different channels. Thus, the inherent information cannot be extracted.

There are known some automated processes for transforming a data set, e.g. the well known parsing facilities used in compilers. These relate, however, to the opposite problem. In the case of a compiler, information that is expressed in a compressed manner in a high level programming language, is expanded to make the program executable for the computer. Functional or structural relations are not added.

Text filters are also known for extracting certain information out of a data set. These basically correspond to the search engines mentioned above and do not add structural information to the input data.

It is the object of the invention to provide a method for structuring a set of signals, especially a set or sequence of data, in a way that more efficient searches can be carried out and facts can be extracted.

This object is accomplished by a method of automatically structuring a set of signals according to predetermined patterns by means of an apparatus for processing said signals, especially a computer, said patterns forming a hierarchy, wherein a pattern of a higher order comprises at least one pattern of a lower order, said method comprising the steps of:
- providing a set of signals comprising at least one unit of signals matching a signal pattern, said unit comprising information identifying the signals comprised in said unit, e.g. data marking the start or end of the unit or indicating the location of elements of the unit,
- comparing a group of signals out of said set of signals, comprising at least one of said units, to one or more predetermined patterns of a higher order than the order of a pattern matching one of said units, especially of an order higher than the highest order of said units contained in said group, in particular the next higher order, by said processing apparatus,
- if a match is found, modifying said set of signals by said processing apparatus, said step of modifying said set comprising the step of replacing the group of signals matching the pattern by a higher order unit corresponding to said group of signals matching said higher order pattern, said unit having an order corresponding to the order of the higher order pattern and comprising information identifying the signals comprised in said unit.

The signals can especially be electronic data in a data format provided for a data processing system. They may, however, also be other physical entities representing information and can especially be transient signals, digital or analogue signals, without necessarily comprising a specific format or shape.

These signals may represent, for example, gene or protein sequences, measurement data, such as atomic spectra, words in an artificial language, such as a programming language, or words in a natural language, just to mention a few possible applications.

Said set of signals can especially be a sequence in time or a sequence according to an imposed order, such as a certain order of storage spaces, but is not restricted thereto. It can also, for example, comprise separate sequences of data taken from a larger entity.

For the sake of clearness it should be mentioned that said group of signals compared to a pattern may also only consist of one signal or signal element. Likewise, said group may, but does not necessarily consist of a coherent sequence of signals. If, for example, the signals represent a spectrum, the peaks related to a certain element may be used as a pattern to compare the signals and accordingly the group of signals chosen for comparison may comprise parts of the spectrum that are separated from each other.

The pattern may especially be a pattern that is not or not exclusively defined by syntactic and/or semantic rules of a natural language.

The term "unit" mentioned previously is understood to mean an element of the structure imposed on the set of signals which is distinguished as a group from other signals in said set of signals, e.g. by appropriate tags. It does not necessarily imply that the unit itself has a certain internal structure, although this may be the case, especially if the unit is a unit of a higher order and comprises units of lower order.

Said step of modifying said group of signals may comprise or consist of adding additional signals into the existing set of signals, e.g. signals marking the start and the end of the unit, so that the group of signals matched is entirely or partly replaced by a group comprising the signals forming the initial group matched by the pattern plus additional signals inserted into said group or part thereof. In other words the group of signals matched or part thereof is enhanced by additional information. Said step may however also consist or comprise the replacement of part or all of the signals of said group by other signals representing the higher order unit. For example, a certain sequence of data having a specific relation between its elements may be replaced by the name of a function with the elements as its arguments.

The invention may provide that one or more units matching a pattern comprise information on the pattern matched.

The invention may especially provide that one or more signals marking the start and/or end of a sequence of said unit are inserted in said initial group matching the pattern. If the group consists of one single coherent sequence of signals, the start and/or end of said group is marked thereby. If said group consists of a plurality of partial sequences, information pointing to the beginning of the next partial sequence may be provided at the end of each partial sequence. Thus, although it is preferred that the unit consists of a sequence of signals between a well defined starting point and a well defined end point, the signals representing the unit need not necessarily be sequential to each other, as long as it is clear which signals belong to the unit and which do not.

The invention may comprise the step of creating additional signals indicating properties of said matching higher order pattern and to assign these additional signals in a retrievable manner to said higher order unit. Said properties of said pattern may especially be properties distinguishing said pattern from other patterns, but may also comprise additional information which may, for example, come in useful in a further search, e.g. comments or explanatory notes by a user, a reference or link to another data set or another unit and the like.

According to the invention, information distinguishing said pattern from other patterns may for example be a property, such as being a noun, if the group of signals matching the pattern represents a word, a physical property, if said group of signals indicates a substance, a certain functionality, if the signals represent a nucleic acid sequence. As another example, said distinguishing information may be a name or another identifier for said group of signals. For a sequence or a spectrum, said information may be a marker marking those parts related to a certain functionality or a certain element.

Said additional information may be introduced into said modified set of signals, as will be explained in more detail. It may however also be contained in a separate set of signals, e.g. a separate set of data, more specifically a separate data file, wherein the entries are correlated with the structural units in a unique and unambiguous manner. Such correlation may be introduced by specific reference data, e.g. links. It may, however, also be inherently contained in said additional separate data, e.g. by structuring these additional separate data in the same or similar manner as the modified set of data.

If additional information on the patterns is provided, one implementation of structuring a sequence of data may, for example, provide that the additional information is stored in a separate reference file and that the first entry in said reference file relates to the pattern of which the data marking the start of the related unit occurs first in the sequence of modified data. In another implementation the unit may comprise data distinguishable from the data of the original data set comprising a reference to a certain entry in the reference file. For example, data referring to the reference file can be distinguishable by way of a certain initial sequence of data, such as e.g. \ref or the like. In a still further embodiment said data may be contained in the modified sequence of data and identified by means of tags marking the start and the end of the additional data, e.g. by "<lemma" marking the start of the additional information and "/lemma>" marking the end of the additional information.

It should be noted that said additional information may be provided not only in the higher order units introduced by the above-mentioned process, but may also be present in or provided for the units contained in the set of signals initially provided.

The processing apparatus may especially be a computer, but can also be any other hardware for processing signals, e.g. implementing a processing algorithm in a hardwired fashion, e.g. in any chip representation. In fact any apparatus which represents a Turing machine can be used to perform the method according to this invention. Any of these apparatus can be used in a cascaded fashion or in a pipeline one after the other.

The set of signals provided for comparison is not necessarily restricted to comprising units of lowest order. Rather, the data set used for comparison to patterns may comprise units of any order lower than the highest order. Especially, it may be the result of a previous structuring step replacing a group of signals by a unit of an order lower than the order of the patterns for which a match is sought..

Matching a group of signals to a pattern does not necessarily mean a 100 % identity. Especially in case of analog data, but also with digital data derived from experiments or from measurements in the real world, there will frequently only be a certain degree of similarity in case of a match. Related matching criteria are well known in the art, e.g. that a suitable metric defined for the signals (e.g. the sum or integral over the difference of subsequent data) yields a distance of the group of signals to the pattern that is less than a predetermined value.

The invention may provide that in case of a non-perfect match of a selected group of signals to a higher order pattern a consistency check is performed as to whether the units of lower order, which are contained in said group of signals matched to the pattern, are consistent with the definition of said higher order pattern and/or if the quality of the match can be improved, if a different assignment of the signals contained in said lower order units to one or more patterns is chosen. For non-consistent units the initial data are restored, i.e. the data identifying the unit and indicating properties thereof are removed, and the process of comparing groups of signals to patterns is repeated, but restricted to the group of signals matching said higher order pattern.

The invention may provide that said step of providing a set of signals comprises:
- providing a set of signals,
- comparing a group of signals forming part of said set to one or more predetermined patterns by means of said processing apparatus,
- if a match is found, modifying or transforming said set of signals by said processing apparatus, said step of modifying said set comprising the step of replacing the group of signals matching the pattern by a unit of signals, said unit having an order corresponding to the order of the matching pattern and comprising information on the signals comprised in said unit.

Again, additional information may be created and stored related to said unit created in said modifying step, which may be contained in said unit, but which may also be contained in a separate set of signals, e.g. a separate data file. This additional information may especially be information distinguishing the pattern matched to said group of signals from other patterns.

Thus, the invention may provide the iteration of the steps of comparing signals to patterns and modifying the signals in case of a match. In one embodiment of the invention, the method starts from a set, e.g. a sequence, of basic data without any structural information and builds up a structure in said data by said comparing and matching steps. At any level, the steps for comparing, matching and modifying parts of the data set are essentially the same, unless indicated otherwise subsequently.

Said step of providing a set of signals may especially comprise or consist of the definition and identification of input signals, especially a sequence of input signals.

The invention may provide that at least one of said patterns to be matched is stored in a database.

Alternatively or in addition the invention may provide that one or more patterns are inherently implemented in the processing means. For example, a program code or a hardwired solution for comparing said signals to a pattern may comprise all necessary steps to verify whether a certain group of signals corresponds to a certain pattern without specifying the pattern in a coherent manner, e. g. without retrieving the definition of said pattern.

The invention may provide that all relevant patterns are stored in a database, that all patterns are implemented in the processing means, especially program code thereof, or that part of the patterns is stored in a database and part is implemented in the processing means.

The invention may also provide that information regarding said patterns is stored in more than one database.

The invention may provide that at one or more levels said step of modifying said set of signals comprises creating a unit of signals which comprises the group of signals matching the pattern as well as additional information indicating the pattern matching said unit.

Such additional information may, for example, be added in the form of attributes or tags in a predetermined data format.

The invention may provide that at one or more levels said step of modifying said set of signals comprises at least partly replacing the original group of signals by new signals representing information related to said pattern.

For example, the group of signals matching the pattern may be replaced by signals representing the name of said pattern or otherwise identifying said pattern. As another example, the invention may provide that if a match involving units of a lower level is made, said modifying step replaces said group of signals by the designation of a function having lower order units as arguments.

The invention may provide that at one or more levels, said step of creating a unit comprises the modification of the set of signals such that at least one pattern, especially a pattern of an order higher than the lowest order, can be searched for and/or extracted.

The invention may provide that at one or more levels, the step of creating a unit comprises inserting searchable information, especially searchable information identifying said pattern.

Said information may e.g. be an identifying group of signals indicating the type of pattern, but may also be a plurality of signals indicating various properties of said pattern, which, taken together, allow for the identification of said pattern.

The invention may provide repeating the steps of comparing a group of signals that have not yet been assigned to a unit at the respective level to one or more patterns and creating a unit in case of a match for those signals.

The invention may provide that if no match is found for a selected group of signals, a new group of signals is selected and compared to said predetermined patterns.

The invention may provide that a group for which no match was found is expanded to comprise additional signals to those contained in the group previously. The invention may provide that signals for which no (expanded) group matching one of said patterns can be found are left unassigned to a unit.

The invention may provide that the steps of selecting a group of signals and comparing it to predetermined patterns are repeated until no further matches to patterns are found at a certain level.

The invention may provide that the steps of selecting, comparing and modifying are repeated at one or more subsequent higher levels, until a level is reached where no match is found or the unit matched to a pattern comprises the entire modified set of signals of the previous level.

According to the first alternative of this embodiment, the structuring process results in a plurality of hierarchical structures, each for a part of the initial set of signals, as there is no common unit on the highest level embracing all information. In the second instance there is a classic hierarchy with one unit at the top and further units depending therefrom.

The invention may provide extracting at least one unit from said set of signals.

Usually this extracting step comprises a search for identifying information in said modified set of data, after the structuring of the data or the structuring up to a certain level has been completed. The invention may also provide that the unit is extracted after a match to a predetermined pattern has been found during said comparing step.

The extracted unit or units may be stored separately from the initial set of signals, e.g. in a database or a file. It may also be displayed on a screen or printed out for display.

The invention also provides an apparatus for automatically structuring a set of signals according to predetermined patterns, said patterns forming a hierarchy, wherein a pattern of a higher order comprises at least one pattern of a lower order, said apparatus performing the following steps when provided with a set of signals comprising at least one unit of signals corresponding to a pattern, said unit comprising information identifying the signals comprised therein:
- comparing a group of signals out of said set of signals, comprising at least one of said units, to one or more predetermined patterns of a higher order than the order of a pattern matching one of said units, especially an order higher than the highest order of one of said units contained in said group, in particular the next higher order,
- if a match is found, modifying said set of signals, said step of modifying said set comprising the step of replacing the group of signals matching the pattern by a higher order unit created from said group of signals matching said higher order pattern, said unit having an order corresponding to the order of the higher order pattern and comprising information on the signals comprised in said unit.

Said unit may also comprise information distinguishing this higher order pattern from other patterns.

The steps performed by said apparatus may especially be steps of any embodiment of a method according to the invention, especially one of the embodiments outlined above.

The apparatus according to the invention may perform the above-mentioned steps of comparing and modifying if provided with any sequence of signals, especially a sequence containing a unit of signals representing a match of a first order pattern and/or a higher order pattern, but also when provided with a set of signals comprising no unit as described above.

The invention also provides a data set, obtainable by a method according to a method of automatically structuring a set of signals as set out above, especially a data set of this kind expressed in a physical medium.

Such medium may be a storage medium, but also an electronic signal used for transmitting information.

The invention may provide that said data set is expressed in a format allowing for the search for one or more patterns corresponding to units in said data set.

The invention also provides a method of searching for patterns in a data set, especially a sequence of data, comprising the following steps:
- providing a data set obtainable by a method as set out above, said data set comprising searchable information assigned to one or more of said units,
- searching for said searchable information.

The invention may provide that said data set is provided with information limited to that of one or more selected searchable units and does not comprise the full information of the initial set of signals. According to this embodiment, part of the initial information was discarded and one or more units were extracted, e.g. to a database or a file.

The invention may, however, also provide that the information in the data set searched is the same as in the initial data set prior to applying the method according to the invention, in which case this information is, however, enhanced by structural information about the patterns present in the data set. Means for extracting one or more units that have been found in a search may, however, be provided.

The invention also provides an apparatus for performing a method of searching for patterns in a data set as set out above.

The invention may especially provide that this apparatus is also able to perform a method of automatically structuring a set of signals, especially a sequence of signals, as set out previously.

Unlike previous parsing techniques, the invention does not map the data onto a new data set having an entirely different structure, e.g. in that a certain storage space is reserved for each structural element, but basically keeps the original sequence of data, to which certain additional data are added, which are distinguished from the the original data. Thus, it is possible to restore the original sequence of data simply by ignoring the additional data added in the process and, given the case, expanding again some functional definitions introduced in the process. It is also possible to use or show only selected ones of said additional data and disregard others in the communication with a user. Thus, the basic structure of the data, e.g. the sequential structure, is maintained.

According to the invention information contained in a set of data, especially a sequence of data, is marked or extracted therefrom by determining whether parts, especially partial sequences, obey predetermined rules.

According to the invention it may be provided that said steps of comparing with a pattern and replacing the matching part involves the identification of a pattern by looking up the pattern or a representative part thereof, e.g. in a reference file or data base. This especially applies to a sequence or a partial sequence of data, which may especially represent a sequence in the biological sense. After identification of the pattern, said sequence may be changed, e.g. by insertion of tags, markers, links or the like, to form a unit comprising the information of the original signals together with additional information. For example, the group of signals matching the pattern may be replaced by a unit which describes the information found and which includes the signals matched or parts of them or a representation of the pattern or of parts thereof. In one embodiment the unit formed thereby forms a new sequence where the additional information was inserted as sequential data. Said additional information may e.g. be the class or standard form. Said unit may also form a combination of one or more sequences in a group, the additional information indicating the sequences that are contained in this group and form part of said unit. The set of signals resulting from said replacement of matching parts by appropriate units can be the input to another step of comparing with a pattern and replacing the matching part where said matching part in particular may contain units introduced in a previous step. Units of higher order are formed thereby, thereby introducing a hierarchical structure in the sequence. The iteration of said step of comparing with a pattern and replacing the matching part forms additional levels in said hierarchy so that there are different hierarchical depths, including a depth zero which comprises signals which were not covered by a match in any step.

As a result of this procedure one obtains one or more hierarchical trees. As the method of the invention works from bottom up, the result may especially be a plurality of trees. This is advantageous in several respects. In many cases, a full hierarchical structure may not even exist. In other cases, errors in the initial data can make it impossible to retrieve the full hierarchical tree. In both cases a system trying to establish a single hierarchical structure for the whole data set will stall. The method of the invention does not use a predetermined hierarchical structure to be matched to the data presented to the processing apparatus, but only matches patterns at a given level in one iteration. Thus it is not necessary to define an entire hierarchy to be matched, but only to define patterns and, given the case, the relation of individual higher order patterns to lower order patterns. The method of the invention is thereby independent to create hierarchical patterns that have not been defined before or accept definitions of patterns that do not fit into known schemes. It may also provide that different hierarchies are defined simultaneously in the same data set, e.g. by labelling patterns and related units by a label for patterns related to each other and performing the matching process for certain patterns related to each other irrespective of previous matches or units established. As, according to one embodiment, the invention merely identifies patterns by adding additional signals or data without deleting the original signals or data, even overlapping patterns, which share common signals or data, may be identified and embodied in the set of signals. The method according to the invention is flexible and allows both for an inherently incomplete hierarchy as well as for errors, in both cases returning partial hierarchies showing the relationship between the data items, as far as they can be established.

The hierarchical structure also makes it possible to extract parts of the information by extracting a node of the tree with all dependent nodes at lower levels, thereby preserving all information relevant to this node (which may be the item searched for) by virtue of the information contained in the lower levels.

The basic principles of the invention are illustrated by a simple example, which is non-limitative and merely intended for the purpose of illustration.

Given an input sequence of signals of the form:
We found that the quick brown fox jumps over the lazy dog."
a lexical analysis, using a dictionary and information on English grammar will provide additional information about the grammatical nature of the various elements separated by blanks in the original sequence (i.e. the words), e.g. as to the type of the word and its state of flexion. The result may be

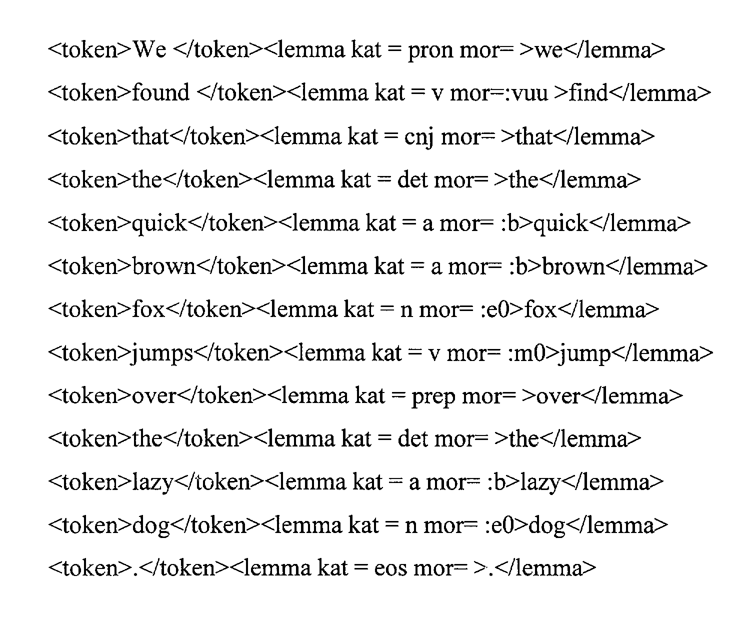

This sequence of data is a unit of the first order in the sense mentioned above. It comprises the initial information plus grammatical information related to the words used.

The next step for establishing units of higher order is to analyse the construction of the sentence. A grammatical database or a grammar checker may provide a rule that if an article and an adjective precede a noun, these form a syntactical unit (noun, prep, adj). Accordingly the system puts in additional information indicating these groups, e.g. as follows: 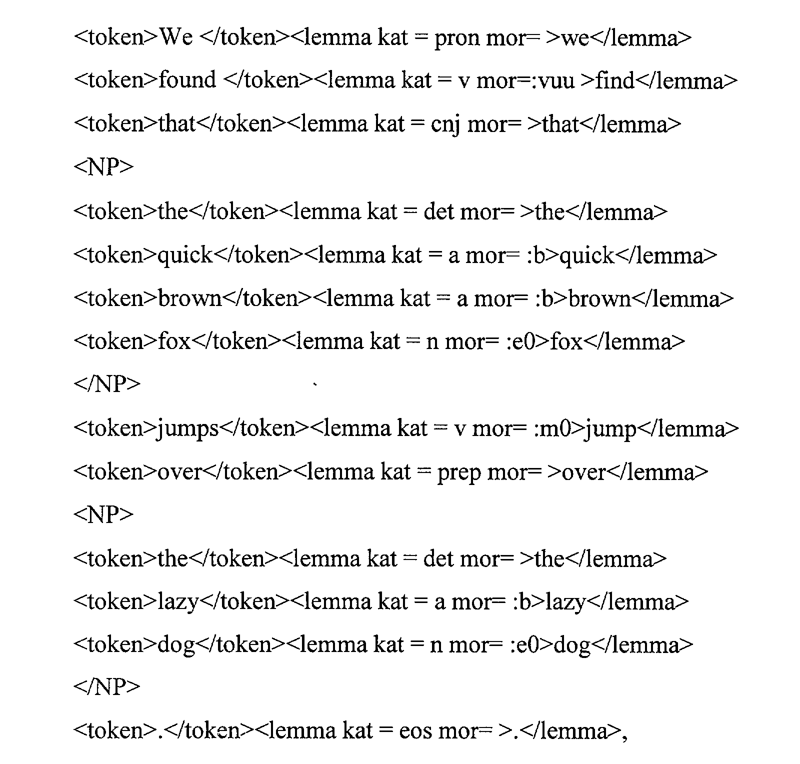 thereby introducing two units (NP-tags) at the second level, namely 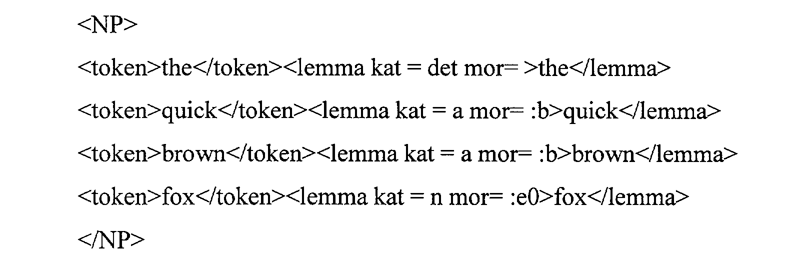 and 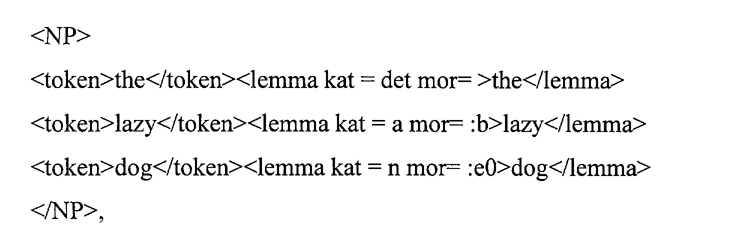 both being marked by markers for the start and the end of the sequence, <NP> and </NP>.

In the next iteration the system applies rules as to the relation of a verb (jump) regarding to its subject and object, resulting in

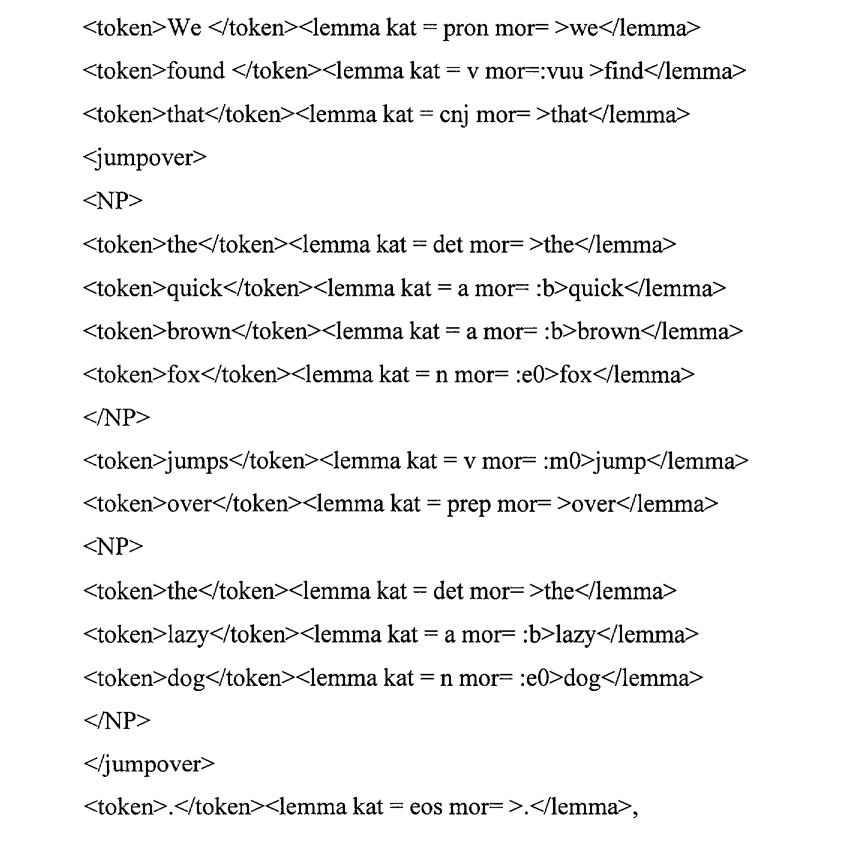

Again, additional data were added in the form of a marker for the start and the end, namely <jumpover> and </jumpover>. It should be noted that additionally identifying information was introduced by the specific name "jumpover", thus making it possible to search for the action jumpover, presuming the question at issue is what jumping actions can be found.

Depending on the purpose of the task at hand and presuming, sticking to the example, that the interest is more in the jumping as in the properties of the one jumping and the one being jumped over, one may compress the information by introducing a function jump_over, resulting in 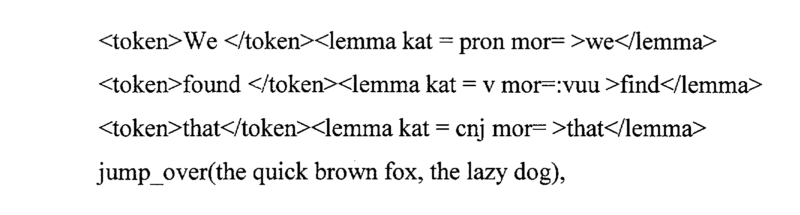 thereby giving up some information that was retrieved earlier on. Thus, the group of data starting from <jumpover> and ending with </jumpover> is removed and replaced by a new group of data derived therefrom. This illustrates the case that the modification of the data set is not effected by introducing additional data, but by replacing certain data by new data. Note, however, that beginning and end of the unit "jump over" is inherently specified by the syntax, e. g. by the rule that a letter immediately following ">" is considered as the beginning of a function and ")" marks the end of a function.

Depending on the requirements one may keep the data in the form indicated above and introduce a search function covering <jumpover> or jump_over, thereby keeping all initial information.

Alternatively (if one does not care who found out who or what was jumping), the function jump_over may be extracted and transferred to another file or another database allowing for a search for jumping actions, simultaneously discarding the following data

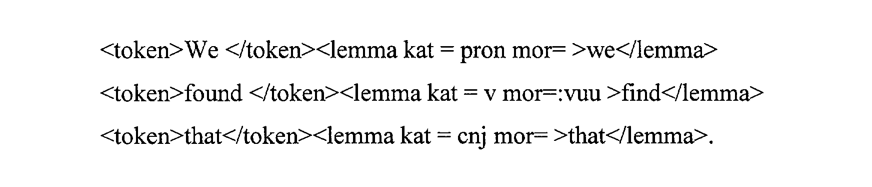

As another example, the application of the invention to biological, especially genetic data will be explained.

Biomolecules consist of sequences of elements, like bases or amino acids. These sequences of single elements can be represented by letters. Thus, the data to be processed and structured will consist of one or more sequences of letters.

Biomolecules have an internal structure comprising so-called domains, said structure embodying the functionality of the biomolecule. Such domains are, for example, exons, introns, coding sequences and GPC - islands in the gene sequence, and alpha-helices, beta-strands, peptides, biased regions and others in the protein. Especially, a combination of three base pairs can represent a triplet encoding one amino acid. Whether a sequence of three base pairs actually encode an amino acid, depends on the region where the triplet is in. Some regions which do not follow this principle may encode a function, e.g. a promotor. A promotor is a characteristic sequence steering a protein which starts to read the DNA.

If a coding region encodes amino acids with base triplets, the entirety of triplets represents a corresponding amino acid sequence.

There are a variety of techniques of identifying such functional domains, e.g. by pattern matching, by software algorithms like BLAST, or by recognition by a scientist.

A possible application of the invention may provide that in a first step entities of three base pairs encoding an amino acid are identified. Each of these triplets is distinguished from the rest of the sequence by introducing a tag marking the beginning of the triplet and a tag marking the end of the triplet. Additional information indicating the amino acid may be added, e.g. by data immediately following the start tag or immediately preceding the end tag and provided with a further tag at the end or beginning thereof, respectively, in order to distinguish it from the data representing the triplet.

On this level an identification of patterns other than nucleotide triplets may also be performed.

In a subsequent iteration, regions comprising triplets encoding amino acids may be identified and regions comprising other domains may be identified and both marked by tags indicating the beginning and the end of such regions. For example, units representing promotors, exons, introns etc., and related units may be created this way. Again, additional information about the nature of this region, if known, is added.

At this level, it may occur that triplets initially identified as encoding an amino acid are found to lie in a non-encoding region. In this case, the process may return one level lower and repeat the pattern matching process for signals within the unit representing said region in order to find a pattern match for those triplets wrongly matched to amino acids.

In a further iteration, further units representing proteins are defined which comprise those units representing the amino acids forming the proteins. Again, beginning and end of these units are marked by tags and an indicator is added marking the unit as corresponding to a protein. Likewise, other known organizational entities are identified and the corresponding data and lower order units are tagged to define a corresponding higher order unit, given the case together with additional information on the organizational entity thus identified. For example, functional relations discovered in research, e.g. the relation of certain domains to diseases, can be embodied by defining a related unit with corresponding tags and corresponding information.

The invention is not limited to text data or genetic data, but can be applied to other data, e.g. a signal representing measurement data, such as spectra, which follow a function embodying certain information. Suitably one will digitize such signals and then insert tags identifying parts of the signal having a certain meaning or function. For example, parts of a measurement curve to be assigned to a unit may be maxima or minima, e.g. defined by their half-widths, the region between two zeros, sections fitted to predetermined functions or defined by a filter function or the like. Higher order units may, for example, identify individual functions that are superimposed in said measurement data, e.g. spectral contributions from different atoms or molecules. For example, if it is known that a certain element provides characteristic peaks in a spectrum at certain positions, in a first iteration the peaks in the spectrum are identified and tagged to form units assigned to these peaks and in a further iteration those peaks characteristic for the element are combined in a further unit assigned to the related element.

Subsequently an example applying the invention to a gene sequence will be given.

This example starts from the following sequence.

aaacgccaat ggtcagattc tcaaaattaa
tttgcatatc gcttgactcc gtacataact
acggaagtaa gcttaagcta tccaaaccaa
atttgaaagg acaagcgtat gtctgaacca
caaaagtctg aaccacaaaa cgggcgcggc
gcgctcttcg ccggt

In a first step single elements are tagged.

aaacgccaat ggtcagattc tcaaaattaa
tttgcatatc gc
<token>ttgact<token><lemma kat="Sig">op35sgn</lemma>
cc gtacataact acggaag
<token>taagct<token><lemma kat="Sig">op10sgn</lemma>
taagcta tccaaaccaa atttgaaagg acaagcgt
<token>atg</token><lemma kat="AA">M</lemma>
<token>tct</token><lemma kat="AA">S</lemma>
<token>gaa</token><lemma kat="AA">E</lemma>
<token>cca</token><lemma kat="AA">P</lemma>
<token>caa</token><lemma kat="AA">Q</lemma>
<token>aag</token><lemma kat="AA">K</lemma>
<token>tct</token><lemma kat="AA">S</lemma>
<token>gaa</token><lemma kat="AA">E</lemma>
<token>cca</token><lemma kat="AA">P</lemma>
<token>caa</token><lemma kat="AA">Q</lemma>
<token>aac</token><lemma kat="AA">N</lemma>
<token>ggg</token><lemma kat="AA">G</lemma>
<token>cgc</token><lemma kat="AA">R</lemma>
<token>ggc</token><lemma kat="AA">G</lemma>
<token>gcg</token><lemma kat="AA">A</lemma>
<token>ctc</token><lemma kat="AA">L</lemma>
<token>ttc</token><lemma kat="AA">F</lemma>
<token>gcc</token><lemma kat="AA">A</lemma>
<token>ggt</token><lemma kat="AA">G</lemma>

One will note that the initial part of the sequence was not assigned to a unit, but a sequence of triplets encoding amino acids was identified (kat = "AA"). The relevant units specify the individual amino acids. Furthermore, two partial sequences were identified which represent a sigma factor binding site (kat = "Sig"). Between these partial sequences and the amino acid triplets there are again partial sequences which were not assigned to a unit at this level.

In a second step, higher order units are identified as follows:

aaacgccaat ggtcagattc tcaaaattaa
tttgcatatc gc
<Operon name="merTPCAD operon">
<token>ttgact<token><lemma kat=Sig>op35sgn</lemma>
cc gtacataact acggaag
<token>taagct<token><lemma kat=Sig>op10sgn</lemma>
</Operon>
taagcta tccaaaccaa atttgaaagg acaagcgt
<Protein name="merT" seq="MSEPQKSEPQNGRGALFAG">
<token>atg</token><lemma kat="AA">M</lemma>
<token>tct</token><lemma kat="AA">S</lemma>
<token>gaa</token><lemma kat="AA">E</lemma>
<token>cca</token><lemma kat="AA">P</lemma>
<token>caa</token><lemma kat="AA">Q</lemma>
<token>aag</token><lemma kat="AA">K</lemma>
<token>tct</token><lemma kat="AA">S</lemma>
<token>gaa</token><lemma kat="AA">E</lemma>
<token>cca</token><lemma kat="AA">P</lemma>
<token>caa</token><lemma kat="AA">Q</lemma>
<token>aac</token><lemma kat="AA">N</lemma>
<token>ggg</token><lemma kat="AA">G</lemma>
<token>cgc</token><lemma kat="AA">R</lemma>
<token>ggc</token><lemma kat="AA">G</lemma>
<token>gcg</token><lemma kat="AA">A</lemma>
<token>ctc</token><lemma kat="AA">L</lemma>
<token>ttc</token><lemma kat="AA">F</lemma>
<token>gcc</token><lemma kat="AA">A</lemma>
<token>ggt</token><lemma kat="AA">G</lemma>
</Protein>

The first two units (sigma factor binding sites) together with the partial sequence between them form an operon which is in fact the merTPCAD - operon.

Furthermore, the sequence of amino acids is combined in a higher order unit representing a protein, namely the protein merT. For reasons of simplicity only a part of the sequence of amino acids of this protein are shown in this example.

In a third step tagging the mercury transporting protein unit together with the regulatory operon could be performed. This is not done in this example, since downstream from the given protein further units can be found, which are not represented to keep this example simple.

This illustrates, how patterns can be identified and sequential data can be structured in biological and especially genetic applications.

The features disclosed in this specification and/or the claims may be material for the realization of the invention in its various embodiments, taken in isolation or in various combinations thereof.

## Claims

1. Method of structuring a set of signals according to predetermined patterns by means of an apparatus for processing said signals, said patterns forming a hierarchy, wherein a pattern of a higher order comprises at least one pattern of a lower order, said method comprising the steps of:
- providing a set of signals comprising at least one unit of signals matching a signal pattern, said unit comprising information identifying the signals comprised in said unit,
- comparing a group of signals out of said set of signals, comprising at least one of said units, to one or more predetermined patterns of a higher order than the order of a unit contained in said group of signals by said processing apparatus,
- if a match is found, modifying said set of signals by said processing apparatus, said step of modifying said set comprising the step of replacing the group of signals matching the pattern by a higher order unit corresponding to said group of signals matching said higher order pattern, said unit having an order corresponding to the order of the higher order pattern and comprising information identifying the signals comprised in said unit.

2. Method according to claim 1, **characterized in that** said step of providing a set of signals comprises:
- providing a set of signals,
- comparing a group of signals forming part of said set to one or more predetermined patterns by means of said processing apparatus,
- if a match is found, modifying said set of signals by said processing apparatus, said step of modifying said set comprising the step of replacing the group of signals matching the pattern by a unit of signals, said unit having an order corresponding to the order of the matching pattern and comprising information on the signals comprised in said unit.

3. Method according to claim 1 or 2, **characterized in that** said unit or units comprise information distinguishing the related pattern from other patterns.

4. Method according to one of claims 1 to 3, **characterized in that** at least one of said patterns to be matched is stored in a database.

5. Method according to one of claims 1 to 4, **characterized in that** at one or more levels of iteration said step of modifying said set of signals comprises creating a unit of signals which comprises the group of signals matching the pattern and additional information indicating the pattern matching said unit.

6. Method according to claim 1 to 5, **characterized in that** at one or more levels of iteration said step of modifying said set of signals comprises at least partly replacing the original group of signals by new signals representing information related to said pattern.

7. Method according to one of claims 1 to 6, **characterized in that** at one or more levels of iteration said step of creating a unit comprises the modification of the set of signals such that at least one pattern can be searched for and/or extracted.

8. Method according to one of claims 1 to 7, **characterized in that** at one or more levels of iteration the step of creating a unit comprises inserting searchable information.

9. Method of one of claims 1 to 8, **characterized by** repeating for those signals that have not yet been assigned to a unit at the respective level the steps of comparing a group of signals to one or more patterns and creating a unit in case of a match.

10. Method according to one of claims 1 to 9, **characterized in that** if no match is found for a selected group of signals, a new group of signals is selected and compared to said predetermined patterns.

11. Method according to one of claims 1 to 10, **characterized by** repeating the steps of selecting a group of signals and comparing it to predetermined patterns until no further matches to patterns are found at a certain level.

12. Method according to claims 1 to 11, **characterized in that** the steps of selecting, comparing and modifying are repeated at one or more subsequent higher levels, until a level is reached where no match is found or the unit matched to a pattern comprises the entire modified set of signals of the previous level.

13. Method according to one of claims 1 to 12, **characterized by** extracting at least one unit from said set of signals.

14. Apparatus for automatically structuring a set of signals according to predetermined patterns, said patterns forming a hierarchy, wherein a pattern of a higher order comprises at least one pattern of a lower order, said apparatus performing the following steps when provided with a set of signals comprising at least one unit of signals matching a signal pattern, said unit comprising information identifying the signals comprised in said unit:
- comparing a group of signals out of said set of signals, comprising at least one of said units, to one or more predetermined patterns of a higher order than the order of a unit contained in said group of signals,
- if a match is found, modifying said set of signals, said step of modifying said set comprising the step of replacing the group of signals matching the pattern by a higher order unit created from said group of signals matching said higher order pattern, said unit having an order corresponding to the order of the higher order pattern and comprising information on the signals comprised in said unit.

15. Data set, obtainable by a method according to a method according to one of claims 1 to 13.

16. Method of searching for patterns in a data set, comprising the following steps:
- providing a data set obtainable by a method according to one of claims 1 to 12, said data set comprising searchable information assigned to one or more of said units,
- searching for said searchable information.

17. Method according to claim 16, **characterized in that** said data set is provided with information reduced to one or more selected searchable units.

18. Apparatus for performing a method according to claim 16 or 17.

19. Apparatus according to claim 18, **characterized in that** it is able to perform a method according to one of claims 1 to 13.
